# EUROPEAN PATENT APPLICATION

(11) **EP 3 988 127 A1**
(43) Date of publication of application: **27.04.2022**
(21) Application number: 20202997.1
(22) Date of filing: 21.10.2020
(51) Int. Cl.: A61L 2/07, A61L 2/00, A61L 2/24

(54) **ARTICULATED ROBOT WITH ELONGATE STEAM HEAD**

(71) Applicant: Weber Hospital Systems B.V., 1689 AP Zwaag (NL)
(72) Inventor: Geelhoed, Cornelis Marinus, 3846 JK Harderwijk (NL); Sierkstra, Erik, 1625 MD Hoorn (NL)
(74) Representative: 't Jong, Bastiaan Jacob

(57) **Abstract**

The invention relates to an articulated robot for disinfecting objects, such as hospital beds, trolleys, wheel chairs and stretchers, which articulated robot comprises:
- a rotational joint arranged at the distal end of the lower arm, which rotational joint comprises an upper joint fork and a lower joint fork rotationally arranged to each other around a rotation axis;
- a twisting joint arranged at the lower joint fork of the rotational joint;
- an elongate steam head arranged at the twisting joint;
- a steam supply line having at least one rotational joint, wherein the rotation axis of the rotational joint of the steam supply line is coaxial with rotation axis of the rotational joint at the lower arm, and wherein the steam supply line has at least one twisting joint, wherein the twisting axis of the twisting joint of the steam supply line is coaxial with the twisting axis of the twisting joint at the lower joint fork.

## Description

The invention relates to an articulated robot for disinfecting objects, such as hospital beds, trolleys, wheel chairs and stretchers, which articulated robot comprises:
- a rotational joint arranged at the distal end of the lower arm, which rotational joint comprises an upper joint fork and a lower joint fork rotationally arranged to each other around a rotation axis;
- a twisting joint arranged at the lower joint fork of the rotational joint.

Such an articulated robot is known in the field for spraying hospital beds with a disinfecting fluid. To this end, a hospital bed is positioned onto a conveyor belt, where the hospital bed is first scanned to determine the type of bed and whether the bed is in a correct position, typically with head and foot part in horizontal position. Then the hospital bed is transported further, such that an articulated robot can spray the bed with a disinfecting fluid.

Basically, this is an automation of hospital bed cleaning performed by people since long. Manually cleaning of a hospital bed is typically done with a sponge or towel and a suitable cleaning agent. Because labor costs rise, the manual cleaning of hospital beds is automated using a robot as described above. The advantage of automated cleaning is the consistency in the quality of the cleaning.

A disadvantage of automatic cleaning is that disinfecting fluid sprayed onto the bed needs to act during some time, then the bed needs to be rinsed with hot water and finally dried before bed sheets can be put again onto the hospital bed. This takes a substantial amount of time per bed.

Another disadvantage is that the used disinfecting fluid is a chemical compound for which strict requirements apply due to environmental impact.

Furthermore, it is known from scientific research that bacteria become resistant against the used chemical compounds over time.

It is an object of the invention to reduce or even remove the above mentioned disadvantages.

This object is achieved according to the invention with an articulated robot according to the preamble, which is characterized by:
- an elongate steam head arranged at the twisting joint;
- a steam supply line having at least one rotational joint, wherein the rotation axis of the rotational joint of the steam supply line is coaxial with rotation axis of the rotational joint at the lower arm, and wherein the steam supply line has at least one twisting joint, wherein the twisting axis of the twisting joint of the steam supply line is coaxial with the twisting axis of the twisting joint at the lower joint fork.

Steam is typically a mixture of water in gas phase and a mist of water droplets. Dry steam has a low amount of water droplets, while wet steam has a high amount of water droplets. Within the scope of this invention, also hot air can be used, which can be considered a very dry steam as air typically contains some amount of water.

Using steam provides an environmental friendly means for disinfecting. Bacteria are killed by the heat of the steam and no chemical compound is required. This also ensures that the bacteria cannot become resistant. Steam also contains little water, such that any condensation dries quickly, such that after disinfecting a hospital bed with steam, it will be almost instantly dry and can be made with bed sheets.

However, a steam head cannot be simply arranged to an articulated robot as any steam hose for providing the steam head with steam will quickly deteriorate due to the many movements of the robot for cleaning a hospital bed and due to the high temperature of the steam, which will harden the inner wall of the hose. This cannot be resolved by providing larger length of steam hose, such that the radius of curvature is decreased, as such larger length will hinder the movement of the robot.

According to the invention, the articulated robot is furthermore provided with a steam supply line having at least one rotational joint, wherein the rotation axis of the rotational joint of the steam supply line is coaxial with rotation axis of the rotational joint at the lower arm, and wherein the steam supply line has at least one twisting joint, wherein the twisting axis of the twisting joint of the steam supply line is coaxial with the twisting axis of the twisting joint at the lower joint fork.

A rotational joint provides rotational relative motion, with the axis of rotation perpendicular to the axes of the input and output links. A twisting joint provides rotary motion, but the axis of rotation is parallel to the axes of the two links.

By arranging a rotational joint and a twisting joint in the supply line and aligning these joints with the rotational joint and twisting joint respectively of the articulated robot, any substantial movement of the steam supply line is prevented and conventional steam hoses can be used.

So, the use of an elongate steam head on an articulate robot in combination with the rotational joint and twisting joint in the steam supply line provides an environmental friendly means for disinfecting objects, such as hospital beds, while ensuring reliability and a long lifespan.

A preferred embodiment of the articulated robot according to the invention further comprises a hose connected with one end to the one rotational joint of the steam supply line and arranged with the other end to a linear guide, which is provided at the proximal end of the lower arm.

In order to further reduce any stress on the steam supply line, a hose can be arranged between the rotational joint and a linear guide, which linear guide will take up any relative movements between the hose and the lower arm of the articulate robot.

Preferably, the elongate steam head comprises steam nozzles arranged in a surface of the elongate steam head, preferably arranged in at least one row.

In a further preferred embodiment of the articulated robot according to the invention the steam nozzles are surrounded by a brush of which the bristles extend substantially perpendicular from the surface in which the steam nozzles are arranged to provide a curtain around the steam nozzles.

The brush will partially contain the steam ejected from the nozzles and direct the steam onto the surface to be disinfected. This provides for a more effective use of the steam, while keeping the elongate steam head at a practical distance from the surface, which ensures that the robot will not collide with the object to be disinfected.

Yet another embodiment of the articulated robot according to the invention further comprising an elongate air supply head, which is arranged parallel to the elongate steam head. With the air supply head any condensation caused by the steam getting into contact with the object to be disinfected can be dried instantly.

The invention further relates to a combination of two articulated robots according to the invention, wherein the work envelope of both robots at least partially coincide.

By having two articulated robots the disinfecting can be done virtually twice as fast. However, by ensuring that the work envelop of both robots at least partially coincide, at least parts of the object, such as a hospital bed, can be disinfected more thoroughly.

The invention further relates to a method for controlling a combination according to the invention wherein both elongate steam heads are positioned parallel to each other and at a distance from each other, and wherein both elongate steam heads are moved along a linear path while being parallel and at a distance from each other.

Especially for disinfecting rod-like elements of the object, the method according to the invention is very well suited. If only a single articulated robot with elongate steam head would be used at a rod-like element, then condensation would be blown at the back of the rod relative to the steam head. Any additional air supply would not be able to evaporate the condensation sufficiently.

By arranging two elongate steam heads opposite of each other with the rod-like element in between, the condensation would not be blown to the backside of the rod, as the other steam head would blow the condensation back. As a result a quick evaporation of the condensation would be achieved.

These and other features of the invention will be elucidated in conjunction with the accompanying drawings.
Figure 1 shows a perspective view of an installation for automatic disinfecting of hospital beds.
Figure 2 shows a perspective view of an embodiment of the articulate robot according to the invention.
Figure 3 shows a detailed perspective view of the steam head of the robot of figure 2.
Figures 4A and 4B show respectively a side view and cross-sectional view of the rotational joint in the steam line of the robot according to figure 2.
Figure 5 shows a detailed perspective view of the robot of figure 2.
Figure 6 shows a combination according to the invention positioned on either side of a bed rail of a hospital bed.

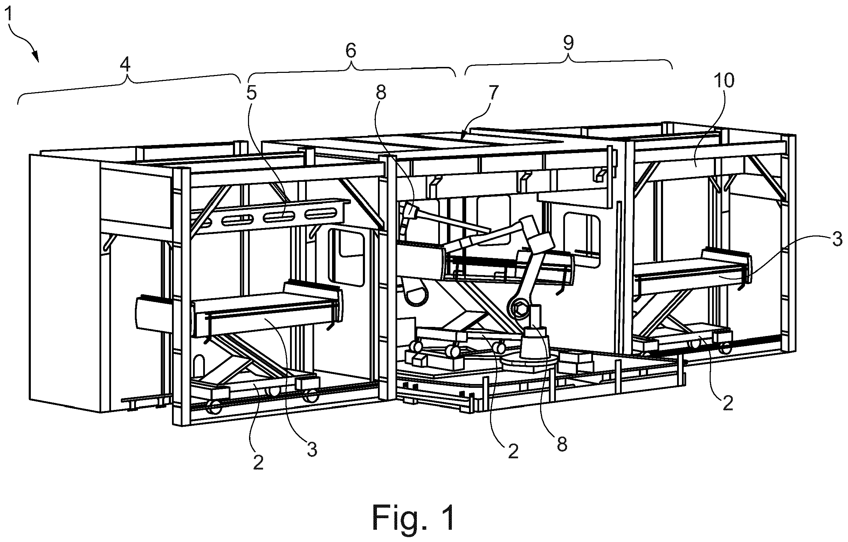

In figure 1 an installation 1 is shown for disinfecting hospital beds 2, 3. The installation 1 has a first zone 4 in which hospital beds 2, 3 are measured to determine which type the bed is and if the bed is in the right position. Then the mattress 3 is lifted off the bed frame 2 by a first lift device 5.

The bed frame 2 is transported to a second zone 6, while the mattress 3 is transported overhead into a device 7 for disinfecting the mattress 3.

In the second zone 6 two articulate robots 8 are positioned, which will clean the bed frame 2 with steam by moving an elongate steam head along the bed frame 2 in a programmed way and depending on the type of bed as detected in the first zone 4.

The mattress 3 is meanwhile disinfected by the device 7 and transported further into the third zone 9. The bed frame 2 is also transported into this third zone 9, such that second lifting means 10 can lower the mattress 3 back onto the bed frame 2.

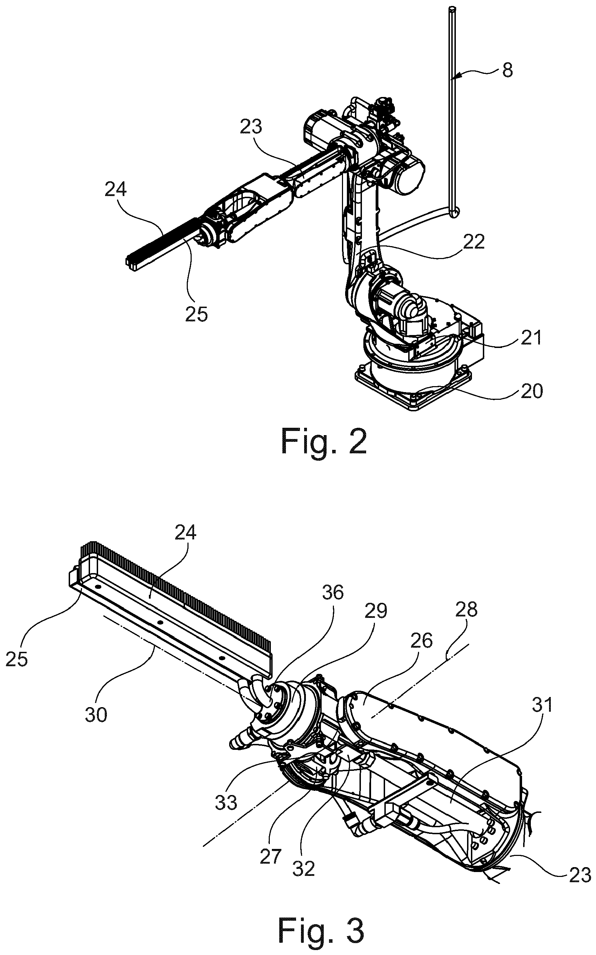

Figure 2 shows the articulated robot 8 in perspective view. The robot 8 has a base 20 on which a shoulder part 21 is rotatably arranged. Rotatably connected to the shoulder part 21 is an upper arm 22 to which a lower arm 23 is rotatably arranged. At the distal end of the lower arm 23 an elongate steam head 24 and elongate air supply head 25 are arranged.

Figure 3 shows the distal end of the lower arm 23, the elongate steam head 24 and elongate air supply head 25 in more detail. The distal end of the lower arm 23 is provided with a rotational joint, which has an upper joint fork 26 and a lower joint fork 27 rotationally arranged to each other around a rotation axis 28. Furthermore, a twisting joint 29 is arranged at the lower joint fork 27 of the rotational joint, which allows for a rotational movement around the rotation axis 30.

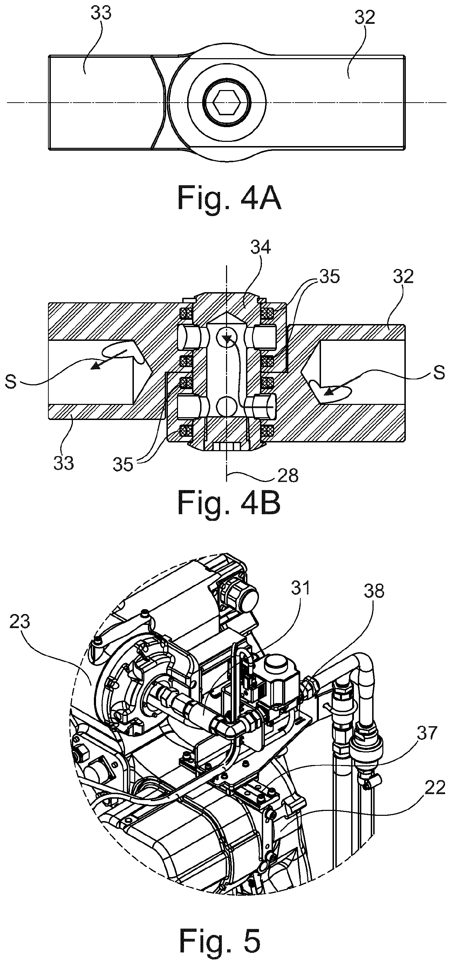

A steam supply line 31 extends through the lower arm 23 and connects to a rotational joint 32, 33 which has a rotation axis coinciding with the rotation axis 28. As shown in figures 4A and 4B the rotational joint 32, 33 has a central, hollow axle 34, which seals be sealing rings 35 to the parts 32, 33 and allows for steam S to flow from the steam supply line 31 towards the elongate steam head 24.

The steam supply line 31 further has a twisting joint 36 coaxially integrated into the twisting joint 29, such that any rotational and twisting movement between the distal end of the lower arm 23 and the elongate steam head 24 can be followed by the steam supply line 31 without causing any stress in the steam supply line 31.

Figure 5 shows a detailed view of the rotational joint between the upper arm 22 and the lower arm 23. The steam supply line 31 runs through the lower arm 23 and is mounted via a bracket 38 on a linear guide 37, which is mounted at the proximal end of the lower arm 23. Any length differences between the distal end and the proximal end of the steam supply line 31 relative to the lower arm 23 can be taken up by the linear guide 37.

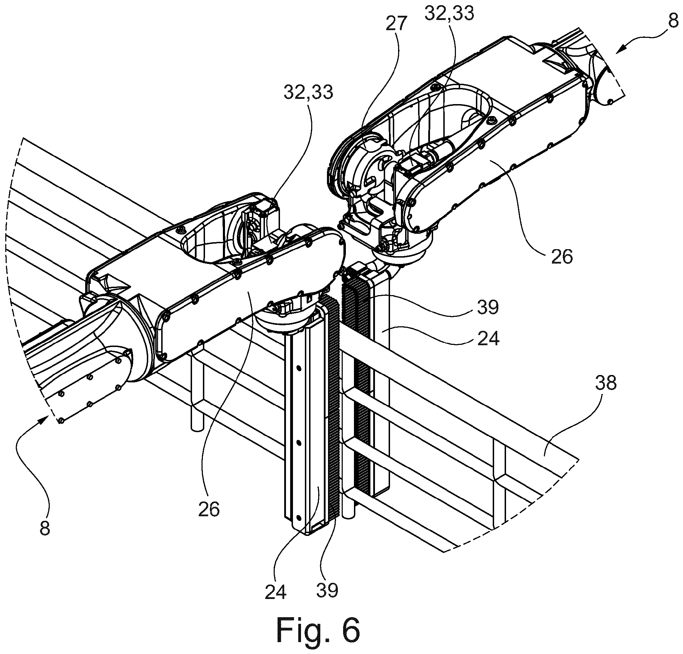

Figure 6 shows a combination of two articulated robots 8, which can be positioned with the elongate steam heads 24 on opposite sides of a bed rail 38 of a bed frame 2. Each elongate steam head 24 is provided with a brush 39, which surround the steam nozzles of the steam head 24. The bristles of the brush direct the steam from the elongate steam head towards the bed rail 38, while the steam head 24 can be kept at a safe distance therefrom. If the bristles of the brush 39 contact the bed rail 38 it will not interrupt the movement of the respective articulated robot 8.

## Claims

1. Articulated robot for disinfecting objects, such as hospital beds, trolleys, wheel chairs and stretchers, which articulated robot comprises:
- a rotational joint arranged at the distal end of the lower arm, which rotational joint comprises an upper joint fork and a lower joint fork rotationally arranged to each other around a rotation axis;
- a twisting joint arranged at the lower joint fork of the rotational joint;
**characterized by**
- an elongate steam head arranged at the twisting joint;
- a steam supply line having at least one rotational joint, wherein the rotation axis of the rotational joint of the steam supply line is coaxial with rotation axis of the rotational joint at the lower arm, and wherein the steam supply line has at least one twisting joint, wherein the twisting axis of the twisting joint of the steam supply line is coaxial with the twisting axis of the twisting joint at the lower joint fork.

2. Articulated robot according to claim 1, further comprising a hose connected with one end to the one rotational joint of the steam supply line and arranged with the other end to a linear guide, which is provided at the proximal end of the lower arm.

3. Articulated robot according to claim 1 or 2, wherein the elongate steam head comprises steam nozzles arranged in a surface of the elongate steam head, preferably arranged in at least one row.

4. Articulated robot according to claim 3, wherein the steam nozzles are surrounded by a brush of which the bristles extend substantially perpendicular from the surface in which the steam nozzles are arranged to provide a curtain around the steam nozzles.

5. Articulated robot according to any of the preceding claims, further comprising an elongate air supply head, which is arranged parallel to the elongate steam head.

6. Combination of two articulated robots according to any of the preceding claims, wherein the work envelope of both robots at least partially coincide.

7. Method for controlling a combination according to claim 6, wherein both elongate steam heads are positioned parallel to each other and at a distance from each other, and wherein both elongate steam heads are moved along a linear path while being parallel and at a distance from each other.
